(19) Europäisches Patentamt / European Patent Office / Office européen des brevets

(11) **EP 4 715 694 A1**

(12) **EUROPEAN PATENT APPLICATION**

(43) Date of publication:
**25.03.2026 Bulletin 2026/13**

(21) Application number: **24202322.4**

(22) Date of filing: **24.09.2024**

(51) International Patent Classification (IPC):
**G06Q 10/04** $^{(2023.01)}$      **G01B 21/08** $^{(2006.01)}$
**G01N 33/46** $^{(2006.01)}$

(52) Cooperative Patent Classification (CPC):
**G06Q 10/04; G01B 21/08; G01N 33/46**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Swiss Wood Solutions AG**
**6460 Altdorf (CH)**

(72) Inventors:
• **Clerc, Gaspard**
**6370 Stans (CH)**
• **Sax, Cédric**
**8037 Zürich (CH)**
• **Sonderegger, Walter**
**8708 Männedorf (CH)**
• **Steiner, Patrick**
**6467 Schattdorf (CH)**
• **Kläusler, Oliver**
**4054 Basel (CH)**

(74) Representative: **Keller Schneider**
**Patent- und Markenanwälte AG**
**Eigerstrasse 2**
**3007 Bern (CH)**

(54) **METHOD FOR PREDICTING A THICKNESS OF AT LEAST ONE WOOD LAYER AFTER DENSIFICATION**

(57)     The invention refers to a method for predicting a thickness of at least one wood layer after densification comprising the steps of:
(i) Determining the surface weight of the at least one wood layer before densification
(ii) Defining a target density for the at least one wood layer after densification
(iii) Predicting the thickness of the at least one wood layer after densification based on the surface weight and the target density

i)

ii)

iii)

**Figure 1**

**Description**

**Technical field**

The invention relates to a method for predicting a thickness of at least one wood layer after densification. Furthermore, the invention is concerned with a method for producing at least one densified wood layer comprising the steps of predicting a thickness of at least one wood layer according to the inventive method and densifying the at least wood layer. Moreover, the invention relates to a method for producing a laminated structure comprising the steps of providing at least two densified wood layers according to the invention and laminating them.

**Background art**

In technical fields, which entail a high consumption of energy and materials from limited natural resources, there is a great and increasing demand for renewable raw materials, such as e.g. raw materials based on plants, for example wood. Renewable materials usually have an advantageous $CO_2$ balance, which is a great advantage when compared to synthetic materials that are usually based on fossil raw materials.

For example, common electronic cards, such as payment cards, including credit cards, debit cards, prepaid cards, guarantee cards, customer cards, identity cards, access cards (e.g. for doors, barriers, check-in terminals etc.) are usually made of plastics such as PVC, PET, polycarbonate, PLA or other polymeric materials. Semi wood cards, with a PVC or PET or other plastic inlays (middle layer) and a top and bottom layer out of non-densified wood material also exist. Such cards have a standardized size of 80 - 90 mm (length) x 50 - 60 mm (width) x 0.65 - 0.85 mm (thickness), as defined in ISO/IEC 7810:2019 under ID-000, ID-1, ID-2, ID-3. Furthermore, also semi- and full metal cards exist. Increasingly, these common electronic cards are nowadays made mainly of natural materials, especially densified natural materials, such as densified wood.

For the manufacturing process of products made of natural materials it is important to know how these materials react structurally and/or chemically to the production process in order to predict the outcome of the final product. For example, for common electronic cards as mentioned above, the final products need to meet standardized requirements, for example with regard to length, width, thickness and stability. However, the methods to date show a lack in preciseness regarding the dimensional requirements of a final product, they are rather complicated to implement and produce a lot of waste.

There is thus a need to provide improved solutions, which overcome the aforementioned drawbacks.

**Disclosure of the invention**

It is an object of the present invention to provide an improved method for simplifying the process of manufacturing products made of densified natural materials, in particular products made of densified wood, as well as to provide a method for reducing the production waste, the expenditures, the production time and the amount of raw materials used.

Surprisingly, it has been found that these objects can be achieved by the features of claim 1. Thus, the core of the invention is a method for predicting a thickness of at least one wood layer after densification comprising the steps of:

(i)      Determining the surface weight of the at least one wood layer before densification
(ii)     Defining a target density for the at least one wood layer after densification
(iii)    Predicting the thickness of the at least one wood layer after densification based on the surface weight and the target density

Knowing that a wood layer before densification has a density $\rho_b$, a surface area $A_b$ and a thickness $t_b$, and a wood layer after densification has a density $\rho_a$, a surface Area $A_a$ and a thickness $t_a$, the thickness of a wood layer after densification ($t_a$) can be expressed as

*Equation 1:*                               $$t_a = t_b \frac{\rho_b}{\rho_a}$$

For wood layers before densification, the density of the wood layer ($\rho_b$) is defined as the ratio of the mass of the wood layer ($m_b$) to the volume of the wood layer ($V_b$), whereby the volume $V_b$ also equals the product of the area of the wood layer ($A_b$) and the thickness of the wood layer ($t_b$) before densification:

*Equation 2:*
$$\rho_b = \frac{m_b}{V_b} = \frac{m_b}{A_b * t_b}$$

By combining equations 1 and 2, the following new equation 3 is obtained:

*Equation 3:*
$$t_a = t_b \frac{\rho_b}{\rho_a} = t_b \frac{m_b}{A_b * t_b * \rho_a}$$

As a result, the thickness of the wood layer before densification can be cancelled out. Since the ratio of the mass of the wood layer ($m_b$) and the area of the wood layer ($A_b$) equal the surface weight of the wood layer before densification ($SW_b$), equation 3 can be written as follows:

*Equation 4:*
$$t_a = \frac{SW_b}{\rho_a}$$

Under the consideration that an inventive densified wood layer has a pre-defined density ($\rho_{a,def.}$), the summed thickness of at least two densified wood layers ($t_{sum,a}$) or n densified wood layers, respectively, can therefore be determined based on the respective pre-defined density ($\rho_{a,def.}$) and surface weight of the at least two wood layers before densification ($SW_{b,i}$) as follows:

$$t_{sum,a} = \frac{1}{\rho_{a,def.}} \sum_{i=1}^{n} SW_{b,i}$$

The pre-defined density of a densified wood layer is described herein also as "target density". The target density is meant to be the density of the at least one wood layer that is achieved after the densification process. The target density is defined before the densification process and the appropriate parameters, for example pressure or temperature, are set such that the target density is achieved.

Furthermore, it has been observed that the target density does not depend on the wood type used for the wood layers. The appropriate parameters that have to be set for the densification process in order to reach the target density may vary for different wood types (e.g. different wood species).

Preferably, the parameters to obtain the target density and a dimensional stability are defined in a calibration procedure. Thereby, for example, a given type of wood can be calibrated with different parameters until the desired target density and dimensional stability is obtained. Thus, the method according to the invention in particular comprises a further step of determining densification parameters to obtain a target density for a given type of wood or for several types of wood. The densification parameters can e.g. include temperature, pressure and/or temperature and pressure profiles.

Based on the findings above, the thickness of at least one wood layer ($t_{sum,a}$) after densification is determined based on, preferably solely based on, the target density ($\rho_{a,def}$) of a densified wood layer and the surface weight of the wood layer(s) before densification ($SW_{b,i}$).

Thus, with the inventive method there is no need to measure the thickness of the wood layer before densification ($t_b$) to determine the thickness of a wood layer, or several wood layers, respectively, after densification ($t_a$).

Known methods require measuring the thickness of a wood layer prior to densification to predict the thickness after densification. However, measuring the thickness of a wood layer can be challenging and imprecise since wood layers often have small thicknesses, e.g. smaller than 0.2 mm, and an uneven surface, too. The inventive approach eliminates the need for measuring the thickness of the wood layer before densification and provides a much simpler, quicker and more precise method of predicting the thickness of a wood layer after densification.

The advantage of the method according to the invention is that the measurement of the surface weight before densification is easy to implement, quick and precise, making the in-line characterization of the material much easier and faster. Furthermore, once the parameters for the process of densification are set, they can be used to produce wood layers with the same target density for a given type of wood. This enhances the production speed of the manufacturing process and lowers the error rate, since less parameter adjustments have to be made.

In a preferred embodiment, the at least one wood layer before densification is densified.

Especially, the wood is selected from any type of angiosperms (hardwoods, flower and fruit bearing, deciduous, e.g. maples, oaks, beech, hickory cherry, walnut) or gymnosperms (softwoods, coniferous, needle-like leaves, evergreen, e.g. firs, spruces, pines) from natural forests or plantations. Furthermore, the part of the tree/wood can be sapwood or heartwood, earlywood or latewood, burls/burrs, roots, leaves and barks.

In a further preferred embodiment, the wood is selected from any type of monocotyledons and/or dicotyledons.

In particular, the wood is selected from any type of monocotyledons, i.e. grass and grass-like flowering plants, especially palms.

The term wood includes native wood as well as modified wood. Wood can e.g. be soft or hard wood. Modified wood stands for chemically and/or physically treated wood. For example, the modified wood can be a wood chemically modified in a gas phase, e.g. in ammonia, wood smoke, and/or another gaseous chemical, or the modified wood is a wood chemically modified in a liquid phase, e.g. acetylated or mineralized wood. Physically modified wood includes for example thermally treated and/or baked wood. Especially, the modified wood is an impregnated wood, in particular with an organic or inorganic compound, e.g. an oil-impregnated wood, or an acetylated wood or a mineralized wood.

Especially, the wood is selected from lumber, in particular solid wood and/or wood veneers.

Lumber stands for wood that has been processed into wood products of a specific form, e.g. into beams, planks and/or veneers. Solid wood can for example be selected from side boards, rift cut, quarter cut. Veneers can be round rotary sliced, half round sliced, plain sliced, quarter sliced, rift sliced, peeled or sawn veneers.

Especially, the at least one wood layer before densification has a thickness of 0.05 - 5.0 mm, especially 0.3 - 1.2 mm, in particular 0.4 - 0.7 mm. However, it is also possible that the thickness is smaller than 0.05 mm or larger than 5.0 mm.

Preferably, the at least one wood layer before densification has a thickness of 0.1 - 4.0 mm, especially 0.3 - 1.2 mm, in particular 0.4 - 0.7 mm. However, it is also possible that the thickness is smaller than 0.1 mm or larger than 4.0 mm.

In a special embodiment, the thickness of the at least one wood layer after densification is 0.01 - 2.0 mm, especially 0.1 - 0.8 mm, in particular 0.15 - 0.3 mm.

In a further preferred embodiment, the at least one wood layer before densification has an area of 0.2 - 1.2 m$^2$, preferably 0.35 - 1.0 m$^2$, especially 0.5 - 0.7 m$^2$.

"Densification" refers to the process of increasing the density of an object, in particular a layer of natural material, especially a wood layer.

In the present context, the expression "wood layer before densification" in particular means "undensified wood layer". Likewise, the expression "wood layer after densification" in particular means "densified wood layer".

Preferably, the densification process comprises the steps of:

a) Providing a wood layer to be densified

b) Optionally, pre-conditioning of the wood layer by adjusting the moisture content of the wood layer to a value within a predefined moisture range, if required;

c) Heating and pressing the wood layer under predefined temperature and pressure conditions, whereby, in particular, the moisture content of the wood layer is kept constant;

d) Obtaining a densified wood layer.

The "moisture content" is defined as the weight of evaporable water contained in a material, e.g. wood, divided by the weight of the material in fully dried state. Put differently, the moisture content is a measure of how much evaporable water is present in the material compared to the weight of the material when all of the evaporable water has been released. The moisture content can be measured with a calibrated moisture meter. Such meters are known to the person skilled in the art. For wood, for example, there are pin-type or pinless-type meters which are commercially available from various suppliers.

According to a further preferred embodiment, in step a), the wood layers to be densified are provided in the form of a stack comprising at least two layers, whereby all layers of the stack are densified together. This approach allows for example for densifying several wood layers simultaneously and/or for producing laminated structures, optionally comprising layers of different wood types.

In particular, the stack comprises at least two layers of different wood types.

In step c), the moisture content preferably is kept constant. This means that the moisture content deviates from the moisture content as possibly adjusted in step b) by a maximum of 10%, especially 5%, in particular 1%. Heating and/or pressing in step c) can e.g. be effected in a mechanical press with a heating device. A heating device can for example be included in a press ram.

In particular, during pressing in step c), the wood layer to be densified can be placed in between a press insert for obtaining a more uniform pressure distribution. The press insert in particular comprises a more elastic material, e.g. rubber, oriented towards the press ram and a less elastic material, e.g. steel, oriented towards the wood layer to be densified. Especially, a press insert is placed on both sides of the wood layer to be densified.

The pressing in step c) can be effected in a static manner or in a continuous manner. Static manner means that in step c), the whole body of the wood layer is treated simultaneously all around. In contrast, continuous manner means that during step c), the wood layer is treated in sections, e.g. by continuously feeding the wood layer into a treatment region or vice versa. Possible feeding rates range from 10$^{-4}$ mm/s to 1 m/s.

In particular, heating and pressing in step c) takes place simultaneously. However, it can also be performed in succession.

The "densified wood layer" as obtained in step d) is a material with a higher density than the density of the wood layer originally provided in step a). Especially, the densified wood layer is a wood layer with compressed pores. Compressed pores are meant to be pores, which have been closed due to the densifying process. This typically results in a surface, which is smoother and/or has a reduced absorption towards liquids when compared with the surface of the wood layer as initially provided. For example, lacquer or other liquid chemicals used to treat the wood layer surface is absorbed less and thus less lacquer is required to treat the surface. Especially, the surface roughness ($Ra_x$) can be reduced to at least half of the roughness of the wood layer before densification.

In particular, the wood layer is transformed to a highly densified wood layer with compressed pores.

The described densification process allows for producing highly densified wood layers with a final density of up to 1'600 kg/m$^3$. Thereby, when compared with the wood layer before densification, it is possible to improve the chemical and physical properties, such as the mechanical stability, colour stability against temperature and UV-Vis light, significantly even without addition of synthetic additives. Thus, highly densified and stable wood layers with and without any synthetic components can be produced.

Dimension stability, hardness, scratch-resistance, colour-stability against ultraviolet (UV) and visible (VIS) light and temperature stability could be improved remarkably in the wood layers by applying the densification process as described above. Furthermore, it is possible to produce translucent wood-based materials, especially without impregnation of any refractive-index matching synthetic polymer, which can transmit light when placed in front of a light source. The translucent properties depend on the wood species, wood fiber and year ring orientation, thickness, natural chemical composition, chemical treatment, degree of densification, and density profile.

The densified wood layers obtained are suitable for laser engraving with no incineration in comparison to natural wood or non-densified wood at a given laser intensity, retention time, feed rate, wood species, wood colour, etc. This allows for customizing the surface structure and/or surface roughness of the densified wood layers, thus allowing for customized haptics and wettability properties, e.g. hydrophobicity or omniphobicity.

The densification process as described above allows for producing densified wood layers with a thickness as low as 0.01 - 0.05 mm. In particular, such kind of wood layers are suitable for producing fully functional cards with integrated electronic functionality. Thereby, it is possible to make cards which comply with the dimensional and mechanical requirements as defined in standard ISO /I EC 7810:2019.

Without being bound by theory, it is believed that because of the heating and pressing of the wood layer under constant moisture conditions, there is no need for steaming of the wood layer during the compression step. Evidently, this results in a densified wood layer in a highly stable state, which is maintained even after releasing the pressure and cooling the wood layer to room temperature. Thereby, the spring-back or shape memory effect of the wood layer, particularly when compared with that one of known densified wood layers (e.g. produced with regular HTM processes), can be reduced significantly. Hence, the described densification process modifies the wood layer structurally and/or chemically in a unique manner.

Furthermore, densified wood layers obtainable with the described densification process are capable of entrapping active agents such as antimicrobial agents or perfume agents, inside the wood structure, thus providing a reservoir of the active agent deep inside the wood, where the active agents is released slowly to the surface either by time (diffusion to surface) or actively upon physical, chemical or mechanical impact such as sawing, cutting, rubbing, scratching, engraving, cleaning with or without a cleaning agent.

Keeping the moisture content constant in step c) can be achieved in different ways.

Especially, the wood layer to be densified is comprised within a self-contained environment during step c). Thereby, the moisture content is kept constant within the environment and thus the moisture content is in the wood layer to be densified does not change significantly.

For example, the device used for heating and/or pressing can be comprised within a self-contained environment and/or the setup of the device used for heating and/or pressing can be designed such that a self-contained environment is realized by machine parts, e.g. press rams, within the device during operation.

According to a preferred implementation of the densification process described above, the process may comprise a further step b1) of gas-tight packaging of the pre-conditioned wood layer in a gas-tight casing before step c).

A "gas-tight casing" is meant to be a covering encasing the wood layer all-around, which is gas-tight for water vapor under the conditions prevailing in step c). In particular, "gas-tight" means a water vapor leakage rate of $\leq 10^{-2}$ mbar*l/s, preferably $\leq 10^{-3}$ mbar*l/s, especially $\leq 10^{-4}$, particularly $\leq 10^{-7}$ mbar*l/s. Especially, the gas-tight casing is a gas-tight wrapping and/or a gas-tight container. Preferably, the gas-tight casing is a foil, a bag and/or a container, which is temperature-resistant under the conditions of step c). In particular, the foil is a plastic, rubber and/or metallic foil. Likewise, the bag and the container can be made of plastic, rubber and/or metal and/or organic polymer. Especially, a container may be compressible.

According to a preferred implementation of the densification process described above, the process may comprise a further step b2) of partly gas-tight packaging of the pre-conditioned wood layer in a gas-tight covering before step c). The covering for example covers at least one side of the wood layer to be densified or the stack.

In a special embodiment, wood is used as a gas-tight casing or covering. Especially, the gas-tight casing or covering is made from a material that is gas tight for water vapor under the conditions prevailing in step c). In particular, "gas-tight" means a water vapor leakage rate of $\leq 10^{-2}$ mbar*l/s, preferably $\leq 10^{-3}$ mbar*l/s, especially $\leq 10^{-4}$, particularly $\leq 10^{-7}$ mbar*l/s.

According to another especially preferred embodiment, at least one outermost layer of the stack forms the gas-tight covering.

This has the advantage that no additional gas-tight casing is needed since the at least one outermost layer itself functions as a gas barrier.

In step b1), the pre-conditioned wood layers can be packaged in the gas-tight casing at atmospheric pressure, at a reduced pressure or at an increased pressure. Thus, "gas-tight packaging" includes methods such as e.g. "vacuum packaging". Thereby, the pre-conditioned wood layer is gas-tight packaged at a pressure below atmospheric pressure.

Vacuum packaging helps to reduce the oxygen content resulting in a less oxidative environment.

According to a preferred embodiment, in step b1), the pre-conditioned wood layer is vacuum packaged, especially with a pressure below atmospheric pressure, in particular ranging between 0.99 bar and $10^{-5}$ bar, preferably $< 10^{-2}$ bar, particularly $< 10^{-3}$ bar.

Gas-tight packaging of the pre-conditioned wood layer in a gas-tight casing is a highly effective and passive measure for keeping the moisture content constant within the wood layer. The tighter the pre-conditioned wood layer is packed, the more constant the moisture content during step c).

However, according to a further preferred implementation of the densification process described above, the process does not comprise a further step of gas-tight packaging of the pre-conditioned wood layer in a gas-tight casing before step c).

In particular, in step c), the wood layer is heated and pressed according to a predefined pressure profile and a predefined temperature profile. A "profile" is meant to be a temporal course of the pressure and the temperature. Put differently, with the predefined pressure profile and the predefined temperature profile, at any point in time during step d), a specific pressure and specific temperature is associated. This helps to control the densifying process in a very precise and reproducible manner.

Especially, for each type of wood layer to be densified, individually determined pressure and temperature profiles are used. Preferably, in step b), the moisture content of the hygroscopic material to be densified is adjusted to 1 - 30%, especially 10 - 15%.

In step c), preferably, the pressure is increased to a value of 5 - 50 MPa, especially 8 - 20 MPa, in particular 10 - 15 MPa and/or the temperature is increased to a value of 100 - 220 °C, especially 120 - 190 °C, in particular 150 - 185 °C. This is in particular suitable if the thickness of the wood layer to be densified is 0.05 - 5.0 mm, especially 0.2 - 1.2 mm, particularly 0.2 - 0.6 mm.

In step c), preferably, the pressure is increased to a value of 5 - 50 MPa, especially 8 - 20 MPa, in particular 10 - 15 MPa and/or the temperature is increased to a value of 100 - 200 °C, especially 120 - 170 °C, in particular 140 - 160 °C. This is in particular suitable if the thickness of the wood layer to be densified is higher than 5.0 mm.

According to a possible implementation, at least one, especially two, in particular all surfaces or the bulk of the wood layer to be densified is chemically treated with a chemical agent before step b), especially by brushing, rolling, spraying, printing, dipping, or impregnation, in particular with a vacuum or pressure treatment.

The chemical agent can be selected from natural polymers, synthetic polymers, biobased polymers, thermosets, thermoplastics, duroplastics, elastomers, natural resins, synthetic resins, waxes, oils, bioactive agents, pharmaceutical active ingredients, colorants, pigments, adhesives, nanoparticles (NPs), microparticles (mRe), clusters and/or agglomerates of NPs and mRe, sulfur, minerals, glasses, ceramics, organo-metal compounds and/or molten or liquid metals. These agents can be liquids, melts, solutions or dispersions. With such a treatment, the densified wood layer can further be adjusted to specific needs. Especially, if there is a chemical treatment, a proportion of the chemical agent is chosen such that a proportion of the chemical agent is from 0.01 - 15 wt. %, especially from 0.1 - 9 wt. %, in particular from 0.5 - 3 wt. %, with respect to the weight of the wood layer to be treated. However, such treatments are only optional.

According to a highly preferred implementation, there is no chemical treatment before step b), especially no impregnation of the surface of the wood layer to be densified. Since the densification process as described results in highly beneficial densified wood layers, there is in general no need to chemically treat the wood layers to be densified.

Especially preferred, step c) comprises the following sub-steps:

c1)    The wood layer is pre-heated to a first temperature at a first pressure and kept for a predefined first dwell time, whereby preferably the first pressure is equal to ambient pressure

(continued)

c2)  While keeping the first temperature, the pressure is raised to a second pressure, especially at a constant rate of pressure increase, and kept for a predefined second dwell time

c3)  While keeping the second pressure, the temperature is increased to a second temperature, especially at a constant rate of temperature increase, and kept for a predefined third dwell time

c4)  Reducing the temperature to room temperature

c5)  Reducing the pressure to ambient pressure

This specific process turned out to be highly beneficial. As is turned out, the independent increase of temperature and pressure in a sequential manner result in highly stable densified wood layers. However, for special wood layers and/or for obtaining special properties in the densified wood layers, a different process control might be beneficial as well.

In another preferred embodiment, step c) comprises the following sub-steps:

c1.1) The wood layer is pre-heated in a first press to a first temperature at a first pressure and kept for a predefined first dwell time

c2.1) While keeping the first temperature, the pressure is raised to a second pressure, especially at a constant rate of pressure increase, and kept for a predefined second dwell time

c3.1) When the wood layer has reached a second temperature, especially at a constant rate of temperature increase, the second pressure and the second temperature are kept for a predefined third dwell time

c4.1) Transferring the wood layer to a second press

c5.1) Cooling the wood layer in the second press to a third temperature, whereby preferably the third temperature is equal to room temperature, at a third pressure for a fourth dwell time.

Especially, the first press is continuously kept at a temperature of 150 °C and/or the second press is continuously kept at room temperature.

In this context, the term "room temperature" means in particular a temperature of 18 - 25 °C.

In particular, in step c1) the first temperature is from 50 - 100 °C, especially 60 - 80 °C, in particular 65 - 75 °C, and the first pressure is from 0-2 MPa, especially 0.1 - 1 MPa, in particular > 0.1 - 1 MPa. 0.1 MPa means atmospheric pressure, i.e. apart from ambient air pressure, no additional pressure is applied. Thus, the pressure as indicated in particular stands for the additional pressure applied by the press device.

Especially, in step c1.1) the first temperature is from 50 - 150 °C, especially 60 - 80 °C, in particular 65 - 75 °C, most preferably 70 °C, and the first pressure is from 0 - 5 MPa, especially 0.1 - 4 MPa, in particular 3 MPa.

Especially, the first dwell time is about 1 min - 10 hours, especially 1 - 60 min, more specially 10 - 20 min and in particular 1-5 min. This is in particular suitable if the thickness of the wood layer is 0.05 - 5.0 mm, especially 0.2 - 1.2 mm, particularly 0.2 - 0.6 mm.

Especially, the first dwell time is about 1 min - 10 hours, especially 5-60 min, in particular 20 min. This is in particular suitable if the thickness of the wood layer to be densified is higher than 5.0 mm.

Especially, in step c2) or c2.1), the second pressure is from 5-50 MPa, especially 9-11 MPa. Thereby, preferably, the constant rate of pressure increase is about 0.5 - 20 MPa/min, especially 5-15 MPa/min, in particular 0.9 - 1.1 MPa/min. In particular, the second dwell time is about 1 - 45 min, especially 20 - 30 min, in particular 1-5 min. This is in particular suitable if the thickness of the wood layer is 0.05 - 5.0 mm, especially 0.2 - 1.2 mm, particularly 0.2 - 0.6 mm.

Especially, in step c2) or c2.1), the second pressure is from 5-50 MPa, especially 9-11 MPa. Thereby, preferably, the constant rate of pressure increase is about 0.5 - 2 MPa/min, especially 0.9 - 1.1 MPa/min. In particular, the second dwell time is about 1 - 45 min, especially 20 - 30 min. This is in particular suitable if the thickness of the wood layer is higher than 5.0 mm.

Especially, in step c2) or c2.1), the second pressure is from 5-50 MPa, especially 9-11 MPa. Thereby, preferably, the constant rate of pressure increase is about 0.5 - 5 MPa/min, especially 0.9 - 1.1 MPa/min. In particular, the second dwell time is about 1 - 10 min, especially 1 - 5 min.

In step c3) or c3.1), in particular, the second temperature is from 100 - 220 °C, especially 120 - 190 °C, preferably between 150- 185 °C, most preferably 150 °C. Thereby, preferably, the constant rate of temperature increase is about 0.5 - 30 °C/min, especially 10-20 °C/min, more especially 5-10 °C/min, in particular 1.5 - 2.5°C/min. The third dwell time preferably is about 1 - 90 min, especially 35 - 55 min, in particular 5-10 min. A fourth dwell time of ca. 1 - 120 min can be added optionally at lower or higher temperatures than the third dwell time. This is in particular suitable if the thickness of the wood layer to be densified is 0.05 - 5.0 mm, especially 0.2 - 1.2 mm, particularly 0.2 - 0.6 mm.

In step c3) or c3.1), in particular, the second temperature is from 130 - 190 °C, preferably between 140 - 160 °C, most preferably 150 °C. Thereby, preferably, the constant rate of temperature increase is about 0.5 - 20 K/min, especially 5-15 K/min, in particular 7.5- 12.5 K/min, mostly preferred 10 K/min. The third dwell time preferably is about 1 - 90 min, especially 35 - 55 min, most preferably 40 min. A fourth dwell time of ca. 10 - 120 min can be added optionally at lower or higher temperatures than the third dwell time. This is in particular suitable if the thickness of the wood layer is higher than 5.0 mm.

In step c4) the temperature is decreased via cooling press rams actively or passively. After the wood layer has cooled down to room temperature, the pressure is released and opened.

Preferably, in step c5.1), the third temperature is from 10 - 35 °C, especially 12 - 30 °C, preferably between 15 - 25 °C, most preferably 20 °C.

Especially, in step c5.1) the third pressure is from 5 - 50 MPa, especially 9-11 MPa, most preferably 10 MPa. Thereby, preferably, the constant rate of pressure decrease is about 0.5 - 20 MPa/min, especially 5-15 MPa/min, in particular 0.9 -1.1 MPa/min, most preferred 1.0 MPa/min. In particular, the fourth dwell time is about 1 - 45 min, especially 20 - 30 min, in particular 1-5 min. This is in particular suitable if the thickness of the wood layer is 0.05 - 5.0 mm, especially 0.2 - 1.2 mm, particularly 0.2 - 0.6 mm.

These parameters described above are especially suitable for densifying wood layers and result in densified wood layers with highly improved properties. Preferably, the densification process further comprises a step of customizing a surface structure and/or a surface texture of the wood material to be densified and/or of the densified wood layer.

In particular, the customization of the surface structure and/or of the surface texture is effected during step c), especially by embossing, e.g. by using a mechanical press with a textured and/or structured press ram and/or by using an embossing insert. An embossing insert can e.g. be made out of metal, Teflon, ceramics, plastic materials, densified wood and/or native wood, being placed on one or both side of the wood layer to be densified.

A special kind of 3D structuration can be achieved, where the structuration corresponds to the wood anatomy, i.e. annual rings, late/early wood profile. For this, two wood layers are superposed and separated with a thin thermo-resistant plastic foil. The two wood layers must come from the same cut in order to have the same wood anatomical features (wood annual rings, late/early wood position etc.). By superposing the two wood layers, both wood layers are pressed simultaneously. As the two wood layers exhibit the same density profile, the low density regions (usually early wood) of both wood layers are densified to higher degree than the high density region (usually late wood), thus creating a 3D surface corresponding to the annual growth rings on the contact surface of both wood layers. By exact positioning of the corresponding early wood regions and the late wood regions of the two wood layers, a 3D structuration of wood surface following the anatomical wood pattern can be achieved. The outer side of both wood layers facing the smooth aluminium plates remain completely smooth and flat, thus yielding one-sided surface structuration. By using 2, 3 or more wood layers, one-sided and double-sided structured wood layers can be obtained.

According to another preferred embodiment, the customization of the surface structure and/or of the surface texture is effected after step c), especially by engraving, e.g. laser engraving and/or CNC-engraving.

However, it is also possible to perform a customization of the surface structure and/or of the surface texture during step c) and/or in addition a further customization after step c). These methods allow for producing densified wood layers with a controlled surface roughness from nano-, over micro- up to millimeter range. Also it is possible to provide hydrophobic, superhydrophobic, omniphobic or even slippery liquid infused porous surfaces (SLIP), when the surfaces are further treated with hydrophobic (oils, fatty acids, waxes) or omniphobic (fluorinated hydrocarbons or perfluorocarbons) agents or any type of hydrophobising coating, lacquer or varnish.

Especially, the customization of the surface structure and/or of the surface texture comprises the step of applying a predefined design, pattern and/or picture onto the surface of the wood layers to be densified and/or of the densified wood layers.

In particular, by the customization of the surface structure and/or of the surface texture, an ultrahydrophobic surface is generated, in particular by micro- and/or nano-structuration, whereby, preferably, the surface has a contact angle with water under standard conditions of 150° or more.

Especially, after step c) or d) at least one layer of the following materials are added to the stack:

1) metals and/or metal alloys, e.g. comprising or consisting of Aluminium, Steel, Copper, Silver, Gold, and/or Titanium; and/or

2) polymeric materials, especially synthetic polymers and/or biopolymers, e.g. PLA, PET, PP, PE, PVC and/or proteins; and/or

3) inorganic materials, e.g. ceramics, glass, stone, clay, metal-organic frameworks (MOF), zeolites, coal, char, carbon black and/or active coal; or other porous or non- porous inorganic materials.

This allows for producing laminated structures or products comprising one, two or more layers of other materials.

The thicknesses and dimensions of the individual layers can vary within wide ranges, depending on the structures or products to be produced. Suitable thicknesses are for example described above.

Especially, non-wood based materials are not densified together with a wood layer.

If, for example, it is a goal to obtain dimensionally stable flat cards, e.g. payment cards and the like, which are according to ISO/IEC 7810:2019 (including ISO/IEC 7811, 7813, 7816 and 14443) and/or similar ones, the densification process as described above is highly beneficial.

In particular, the densification process allows for a targeted combination of layers after step c), e.g. 2 - 48 layers in total, of individual layers of anisotropic materials, e.g. wood, and/or layers of isotropic materials, e.g. paper, fleece, PLA, Protein film etc., with various thicknesses, e.g. ranging from 10 $\mu$m to 420 $\mu$m per layer, such that a laminated structure and/or card with high dimensional stability, suitable dimensions and proper positioning of electronic and/or magnetic functionalities, e.g. an antenna and chip module, are obtainable.

Thereby, anisotropic forces induced by the environmental conditions can be balanced by choosing the right type, number and thickness of the individual layers in the stack.

Specifically, it is possible to produce laminated structures and/or cards that essentially keep their shapes, size and flatness under varying environmental conditions. Put differently, such kind of laminated structures and/or cards are substantially insensitive to varying humidity, wetness, temperature and/or pressure conditions.

Therefore, according to a preferred embodiment, the type, number and thickness of the individual layers in the stack are chosen such that a laminated structure is obtained that can be used for producing a card with mechanical properties according to ISO/ 1 EC 7810:2019 (including ISO/IEC 781 1, 7813, 7816 and 14443).

Preferably, the outermost layers in the stack are made from lumber, in particular solid wood and/or wood veneers.

In particular, at least one of layer of the stack is treated with an adhesive, especially a synthetic, natural, fossil or bio-based adhesive, and/or at least one adhesive foil is arranged between the layers of the stack, such that all the layers of the stack are bonded together upon densification in order to produce a laminated structure.

For example, in between neighbouring layers, adhesive and/or an adhesive foil is placed. In this manner, all of the layers of the stack are bonded together in the densification process.

Especially, the stack comprises an electronic functionality, a magnetic functionality, and/or an optical label. In particular, the electronic functionality is an integrated circuit, a memory device, a tracking device, a sensing device, an electrical wire, an antenna, a capacitive entity and/or an electromagnetic coil. Such kind of electronic functionalities can be included in the stack for producing products with electronic function. However, most preferably, the electronic functionality is selected from an electrical wire, an antenna, a capacitive entity and/or an electromagnetic coil. The functionalities are less temperature sensitive.

Other functionalities can e.g. be added to the stack after densification, as described below. In particular, the electronic functionality, the magnetic functionality, and/or the optical label is arranged on a layer of cellulosic material, especially on a layer of paper. This allows for arranging the functionality or the label, respectively with a well-defined orientation and in space saving manner. However, other arrangements of the functionalities are possible as well.

In particular, all of the layers in the stack have a same length and width. This allows for producing cuboid laminated structures or products.

However, the in another preferred implementation, the layers in the stack have different lengths and/or widths. This allows for producing two- or three dimensional structures with essentially any kind of shape.

Especially, at least two layers of the stack, especially the outermost layers of the stack, are made of wood. According to a further preferred implementation, at least two layers of the stack are made of wood, whereby the layers are oriented with different wood grain directions. Especially, all adjacent layers have a different grain direction. These configurations can be used to obtain laminated structures or products with improved properties, e.g. improved mechanical properties.

For example an angle between the grain directions of the at least two layers oriented with different grain directions, especially between the adjacent layers, is between 0 and 360°, especially 1° - 359°, in particular 30°-45°, especially 60 - 90°.

Especially, the stack comprises at least three layers, in particular at least four layers, whereby, preferably, a thickness of the outermost layers of the at least three layers is larger than a thickness of the one or more layers located in between the outermost layers. Such configurations are especially suitable for producing cards.

In another preferred implementation, the stack comprises at least three layers, in particular at least four layers, whereby, preferably, a thickness of the outermost layers of the at least three layers is smaller than a thickness of the one or more layers located in between the outermost layers. According to another preferred embodiment, a surface structure and/or a surface texture of at least one outermost layer of the at least two layers of the stack is customized during step c), especially by embossing, e.g. by using a mechanical press with a textured and/or structured press ram and/or by using an embossing insert. As described above, an embossing insert can e.g. be made out of metal, Teflon, ceramics, plastic materials, densified wood and/or native wood, being placed on the at least one outermost layer one.

Especially the densification process is effected such that at least one of the at least two wood layers in the stack is rendered translucent. In particular, more than one or all wood layers in the stack are rendered translucent. Translucency of the wood layers can be controlled by the thickness, wood type and lignin content of the wood layers. For example, lignin rich wood layers easily can be rendered translucent in the densification process.

In a further preferred implementation, after step d), a recess and/or an engraving is produced at least in one outermost layer of the stack, whereby, preferably, the recess and/or the engraving is produced by laser-engraving and/or laser cutting. This allows for example for providing a receiving area for further elements, e.g. electronic functionalities, and/or for labelling and/or structuring the at least one outermost layer.

Especially, after step d), an electronic functionality, a magnetic functionality, and/or an optical label is placed in the recess, for example by adhesively bonding. In particular, the functionality is a functionality as described above, especially selected from an integrated circuit, a memory device, a tracking device, a sensing device, and/or chip.

Thereby, preferably, an electronic functionality is arranged such that it is in electrical contact with the other electrical functionality, especially an electrical wire, in particular an antenna and/or an electromagnetic coil, already comprised in the stack. Especially, the recess and/or the engraving is produced such that the layer lying directly under the at least one outermost layer is exposed. In particular, the layer lying directly under the outermost layer is a layer with a colour different than the colour of the outermost layer. This allows for example for producing a logo, letters and/or numbers in a colour different from the colour of the at least one outermost layer, e.g. with higher contrast. There is no need of a separate colouring process.

For example, after step d) the outermost layer is a densified wood layer and the layer lying directly under the outermost layer is a layer of metal and/or plastics, e.g. with a golden, silver or copper color.

Furthermore, after step d), the densified stack can be cut to size and/or cut into several individual densified stacks, especially by laser cutting, knives, punch or other of type of cutters. This allows for example for obtaining several individual stacks or laminated structures, e.g. for cards, with only one single densification step. Therefore, such an approach is highly beneficial for efficient mass production.

The densification process can be part of a process for producing a laminated structure or implemented as a process for producing a laminated structure.

A laminated structure can be directly produced when the wood layer to be densified is provided in the form of a stack, as described above. Thereby, the laminated structure is produced during densification in step c).

Another possibility for producing a laminated structure is described in the following: For producing a laminated structure, especially a laminated wood structure, wood layers or a stack as defined above is densified with the densification process as described above and, afterwards, laminated with at least one further layer, e.g. by adhesively bonding. Thereby, the at least one further layer is for example made of:

1.1) another wood layer densified with the densification process as described above; and/or

1.2) wood-based material, especially paper, plant and/or wood fibers, wood-chips, wood flour, cellulose, starch, lignin, wood bark, wood extractives, native wood; and/or

1.3) metals and/or metal alloys, e.g. Aluminium, Steel, Copper, Silver, Gold, and/or Titanium; and/or

1.4) polymeric materials, especially synthetic polymers and/or biopolymers, e.g. PLA, PET, PP, PE, PVC and/or proteins; and/or

1.5) inorganic materials, e.g. ceramics, glass, stone, clay, metal-organic frameworks (MOF), zeolites, coal, char, carbon black and/or active coal; or other porous or non- porous inorganic materials.

In this production of a laminated structure, the laminated structure, at least in parts, is produced after densification.

Especially, the wood layer densified with the densification method mentioned under item 1.1) is a layer of translucent material. In particular, more than one or all layers in the stack are translucent layers.

If, before laminating, there is a recess produced in the wood layer or in at least one outermost layer of the stack, an electronic functionality and/or a magnetic functionality can be placed in the recess before laminating, such that upon lamination, the electronic functionality and/or magnetic functionality is embedded within the laminated structure. This allows for example for producing laminated structures or products with fully integrated electronic functions, such as e.g. chips, e.g. NFC, RFID, or UHF-RFID chips. Especially preferred, the laminated structure is produced such that the laminated structure comprises:

2.1) a backside made of a layer of a densified or non-densified wood veneer

2.2) a frontside made of a layer of a densified or non-densified wood veneer

2.3) optionally, one or more further layers of a densified or non-densified wood veneer, or any non-wood-based material, which are arranged between the backside layer and the frontside layer

2.4) an integrated circuit, a memory device, a tracking device, a sensing device, an antenna and/or an electromagnetic coil, preferably embedded in the card

2.5) optionally, a support layer, especially made of cellulosic material, e.g. paper, densified wood, and/or fleece, which is arranged between the backside and the frontside, for carrying one or more of the integrated circuit, the memory device, the tracking device, the sensing device the antenna and/or the electromagnetic coil

whereby the layers are laminated and at least one of the layers of wood veneer, in particular the frontside and the backside layers, especially all of the layer of wood veneer, are made of densified wood veneer.

A laminated structure with such a configuration is highly suitable for cards, especially a payment card, a credit card, a debit card, an identity card, a member card and/or an access card.

Thereby, preferably, a thickness of the frontside and the backside is larger than a thickness of the one or more optional further layers.

Furthermore, the process of producing a laminated structure can be part of a process for producing a product or implemented as a process for producing a product.

Thus, a further aspect of the invention is concerned with a method for producing a product comprising the steps of

a1) densifying a wood layer or a stack as described above and/or producing a laminated structure as described above, and

a2) manufacturing the product at least partly from the densified wood layer and/or the laminated structure obtained in step a1).

Thereby, the product in particular is a musical instrument or a part of it, a furniture, a door, a door handle, a floor covering, a wall covering, a revetment, an automotive part, a covering for a ceiling, a sports equipment, a load-bearing element, a card, an electronic device, or a casing for an electronic device, e.g. a key fob, a data storage device, for example a USB stick, or a casing for a mobile phone.

For example, an electronic device can have any shape, e.g. card-shaped, cuboid, sphere shaped, or ring-shaped. The electronic device can feature any kind of electronic functionality, e.g. a sensor function, an access tag or function, identity function, an audio and/or display function.

Especially the electronic device is a wearable, i.e. a computing device worn on the body. For example, the wearable is configured as a finger ring, a bracelet, a protective sports equipment, a watch, or as glasses.

Especially, the product comprises an identification tag and/or a security tag, in particular comprised within the product and/or placed on a surface of the product.

For example, the product comprises a hologram and/or a magnetic strip on the surface of the product and/or the product comprises radiopaque metal particles and/or a chip embedded within the product. These features can serve as security and/or identification tag.

According to a preferred embodiment, above-mentioned electronic functionalities, magnetic functionalities, optical labels, identification tags and/or a security tags can be embedded within the product and/or placed on a surface of the product during the inventive method and/or later on.

Most preferred, the product is a card, especially with electronic functionality, comprising at least one layer of a densified wood veneer as described above.

Especially, the product is a card, especially a payment card, a credit card, a debit card, an identity card, a member card and/or an access card.

Especially preferred, the card is produced such that it comprises:

3.1) a backside made of a layer of a densified or non-densified wood veneer

3.2) a frontside made of a layer of a densified or non-densified wood veneer

3.3) optionally, one or more further layers of a densified or non-densified wood veneer, or any non-wood-based material, which are arranged between the backside layer and the frontside layer

(continued)

3.4) an integrated circuit, a memory device, a tracking device, a sensing device, an antenna and/or an electromagnetic coil, preferably embedded in the card e) optionally, a support layer, especially made of cellulosic material, e.g. paper,

densified wood and/or fleece, which is arranged between the backside and the frontside, for carrying one or more of the integrated circuit, the memory device, the tracking device, the sensing device, the antenna and-/or the electromagnetic coil,

whereby the layers are laminated and at least one of the layers of wood veneer, in particular the frontside and the backside layers, especially all of the layer of wood veneer, are made of densified wood veneer.

Especially, the card fulfills the requirements of ISO /I EC 7810:2019 (including ISO /I EC 781 1, 7813, 7816 and 14443).

A card is meant to be a flat body whereby a length and a width of the card is at least 10 times, especially at least 20 times, preferably at least 50 times, longer than its thickness.

Further beneficial features of densified wood layers, products and cards are described below. Thus, the methods as described above preferably are implemented such these features result.

In particular the card has an overall thickness of 0.5 - 2.5 mm, especially 0.6 - 1.5 mm, preferably 0.7 - 0.9 mm or 0.8 mm. Preferably, the card has a size of 80 - 100 mm x 50 - 70 mm x 0.5 - 1.5 mm, in particular a size of 90 mm x 60 mm x 0.8 mm.

In particular, at least one layer of the densified wood veneer, especially all layers of wood veneers, has/have the same length and width as the card.

Especially, a backside and a front side of the card each are made of a layer of a wood veneer, especially a densified wood veneer as described above.

With respect to the overall weight of the card, the card preferably consists to an extent of at least 40 wt. %, in particular at least 50 wt. %, especially at least 75 wt. % or 95 wt. % or 99 wt. %, of wooden material, especially of densified wood as described above.

A further aspect of the present invention is related to a densified wood layer obtainable or obtained by the method as described above.

Especially, the above described methods are implemented such that densified wood layers with properties as described in the following result.

According to a preferred embodiment, the card comprises at least two layers of laminated wood veneer, whereby, preferably, both of the at least to layers of wood veneer are densified wood veneers as described above.

Especially, at least two of the at least two layers of laminated wood veneer are oriented with different wood grain directions. Especially, all adjacent layers of laminated wood veneer have a different grain direction.

For example an angle between the grain directions of the at least two layers, especially between the adjacent layers, of laminated wood veneer is between 0 - 360°, especially 1 - 359°, in particular 30°-45°, especially 60 - 90°.

With such an arrangement, the mechanical properties and stability of the card can be improved significantly. This will give rise to a more homogenous mechanical stability as the stiffness in the longitudinal and traversal axis of the card are similar.

In particular, each of the layers of wood veneer has a thickness of 0.1 - 0.3 mm. With such thin layers, it is possible to provide the card in the form of a laminated structure with several layers of wood veneers resulting in further improved mechanical properties.

Preferably, the at least two layers of laminated wood veneer are bonded together and/or with other layers of the card with an adhesive. This allows for a very uniform interconnection between the layers of the card.

According to a special embodiment, the card comprises an integrated circuit (IC), memory device, a tracking device, a sensing device, an antenna and/or an electromagnetic coil, especially embedded within the card and/or placed on a surface of the card. For example, the card can have one or more of the following functionalities: radio-frequency identification (RFID) (e.g. 125 KHz, 860-960 MHz, 13.56 MHz or other frequencies), near-field communication (NFC), data storage and/or energy harvesting functionality.

With these components, it is possible to realize cards with electronic functionality such as required by payment cards, access cards, member cards and the like.

Especially, the integrated circuit (IC), memory device, the tracking device, the sensing device antenna and/or electromagnetic coil, if present, are placed in a recess of the at least one layer of densified wood veneer. With such an arrangement, the whole electronic functionality can be included inside the card without any protruding elements.

A further subject of invention is a core-layer for a card. The core-layer is also called inlay for a card. Especially, the inlay is a support layer made of densified wood carrying the electronic functionalities as described above, e.g. an integrated circuit, a tracking device, a sensing device, an antenna, an electromagnetic or inductive coil, an electronic chip, a transistor, a diode, a die, a module and/or CPU, etc.

In a preferred embodiment, electronic entities can be integrated into or onto the densified wood layers after the densification process by methods such as gluing, pressing in or stitching, metal wires or printing conductive metal inks (inkjet, drop on demand), yielding highly densified wood layers with electronic functions and functionalities, such as the electronic inlays for the electronic wood cards.

According to a further preferred embodiment, at a card surface, a pattern of metal contacts for establishing an electrical connection to the integrated circuit, tracking device, sensing device, memory device, antenna and/or electromagnetic coil is arranged. With such contacts it is possible to establish a wired connection to one or more of the electronic components of the card in a suitable reader. However, it is possible to provide a card without any contacts, if required.

Especially, a card surface comprises an engraving, in particular in the form of a logo, letters and/or numbers. Thereby, preferably, the engraving is coated with a color that is different from the color of the surrounding area. In particular, the engraving is obtained by laser engraving.

For example, the engraving can represent personal, legal and/or commercial data, including a name of a holder of the card, a name of the issuer of the card, a member name, a number of the card, a number of a bank account, a logo of the issuer of the card, promotional information, a practical advice, security information, legal information, instructions and the like. Moreover, the densified wood surfaces, especially card surfaces, can be directly printed with common inkjet printers, e.g. based on continuous inkjet, drop-on-demand or bubble-jet techniques).

Furthermore, if desired, the card may be coated with a transparent coating, e.g. a wax or a lacquer.

A highly preferable card comprises:

4.1) a backside made of a layer of a densified or non-densified wood veneer

4.2) a frontside made of a layer of a densified or non-densified wood veneer

4.3) optionally, one or more further layers of a densified wood veneer, non-densified veneer or any non-wood-based material, which are arranged between the backside layer and the frontside layer

4.4) an integrated circuit, a memory device, a tracking device, a sensing device, an antenna and/or an electromagnetic coil, preferably embedded in the card

4.5) optionally a support layer, e.g. a plastic layer, a layer of cellulosic material, a layer of densified wood veneer, which is arranged between the backside and the frontside, for carrying one or more of the integrated circuit, the memory device, the tracking device, the sensing device, the antenna and/or the electromagnetic coil,

whereby the layers are laminated together and at least one of the layers of wood veneer, in particular the frontside and the backside layers, especially all of the layer of wood veneer, are made of densified wood veneer as described above.

Especially, the card fulfills the requirements defined in standard ISO/ 1 EC 7810:2019.

Preferably, the densified material is densified solid wood and/or densified wood veneer. In particular, the wood is selected from any type of angiosperms (hardwoods, flower and fruit bearing, deciduous, e.g. maples, oaks, beech, hickory cherry, walnut) or gymnosperms (softwoods, coniferous, needle-like leaves, evergreen, e.g. firs, spruces, pines) from natural forests or plantations. Furthermore, the part of the tree/wood can be sapwood or heartwood, earlywood or latewood, burls/burrs, roots, leaves and barks.

In particular, the densified wood layer, in particular a wood veneer, has a thickness of 0.05 - 2 mm, especially 0.1 - 1 mm, in particular 0.15 - 0.3 mm.

According to a highly preferred embodiment, the obtainable or obtained densified wood layer is free of additional components, especially free of additional natural polymers, synthetic polymers, natural resins, synthetic resins, waxes, sulfur, and/or molten metals. In this case, the densified wood layer is purely based on the wood as such. "Free of" means that a proportion of the additional components is below 1 wt. %, especially below 0.1 wt. % or 0 wt. %.

Especially, the obtainable or obtained densified material is a translucent material, in particular a translucent wood material. Translucency of the wood materials can be controlled by the thickness, wood type and lignin content of the wood material. For example, lignin rich wood becomes translucent in the inventive method. Thus, the densification preferably is effected such that the wood layer to be densified is rendered translucent.

In particular, for the densified wood layer, the tensile modulus or young's modulus, respectively, is 10'000 - 50'000 MPa, especially 12'000 - 40'000 MPa, in particular 15'000 - 35'000 MPa. This is in particular true for wood of type oak, maple and birch. The tensile modulus defines the relationship between stress and strain in the material in the linear elasticity regime of a uniaxial deformation in the longitudinal direction.

Preferably, for the densified wood layer the density of the densified wood ranges from 900 - 1'600 kg/m$^3$, especially 1'200 - 1'600 kg/m$^3$.

A further aspect of the present invention is a laminated structure comprising at least two laminated layers, especially a laminated wood structure, whereby at least one, especially at least two, of the laminated layers are made of the densified wood layers as described above and/or the laminated structure is obtainable or obtained by the method as described above. Thereby, preferably, the densified wood layer is present in the form of a densified wood veneer.

Especially, the above described methods are implemented such that laminated structures with properties as described in the following result.

Especially, each wood layer can vary in thickness, wood species, wood fiber orientation, degree of density, degree of roughness/smoothness, relative wood fiber orientation (relative angles from one layer to another layer) of the different wood layers. Also a laminating adhesive can vary in its type, chemical composition, dry thickness, origin (synthetic, natural, fossil or bio-based) and physical, chemical and mechanical properties and application technique (such as casting, dipping, brushing, spraying or inlaying of the glue in liquid, gelatinous or solid (film, foil) form.

In particular, the laminated structure comprises two, three, four, five, six, seven, eight, nine, ten or more laminated layers. Thereby, preferably, all of the laminated layers are made of the densified material as described above, especially of densified wood veneer. A combination of densified and non-densified material in the same laminated wood structure is also possible.

Preferably, the layers of the laminated structure are firmly bonded, e.g. with an adhesive and/or an adhesive foil.

Especially, in the laminated structure at least one layer of a densified wood layer as described above is combined with one or more other layers, e.g. layers of non-densified materials. However, most preferred, all of the layers of the laminated structure consist of densified wood layers as described above.

For example, in the laminated structure at least one layer of a densified wood veneer as described above is combined with one or more other layers, e.g. layer(s) of another wood veneer, e.g. a non-densified wood veneer.

However, most preferred, all of the layers of the laminated structure consist of densified wood veneers as described above. Such kind of laminated structures can be used to obtain materials with improved properties, e.g. improved mechanical properties.

Especially, if the materials of the individual layers in the laminated structure have a grain direction, such as in a laminated wood structure, for example, at least two layers of laminated structure are oriented with different grain directions.

Especially, all adjacent layers in the laminated structure have a different grain direction.

For example an angle between the grain directions of the at least two layers oriented with different grain directions, especially between the adjacent layers, is between 0 - 360°, especially 1° - 359°, in particular 30°-45°, especially 60 - 90°.

Preferably, the surface weight is determined by weighing the at least one wood layer and measuring the area of the at least one wood layer, both before densification.

The weighing is preferably conducted with a weighing machine, for example a precision scale.

The area is preferably measured with a device for recording visual images in the form of photograph, film and/or video signals, in particular a camera. Especially, the device for recording visual images encompasses an image sensor which captures the light flowing through a lens of the device creating pixels as a result. The pixels are then used to determine the area of the at least one wood layer based on colour or grey value differences. Most preferred, a calibration is carried out to convert the measured number of pixels into a metric unit.

This has the advantage that the surface weight and the area of the at least one wood layer can be determined in a time efficient and simple manner.

However, it is also possible, that the surface weight and the area of the at least one wood layer are determined with other methods. For example, the area may also be measured with a ruler or a measuring tape.

In a special embodiment, the area of the at least one wood layer before densification is measured with a camera.

This has the advantage that the area can be measured very precisely, easily and quickly, for example based on a fixed image. Furthermore, the visual images taken by the camera may be stored for reviewing purposes.

Nevertheless, it is also possible that the area of the at least one wood layer before densification is measured with other devise, for example with a ruler or a measuring tape.

In a preferred embodiment, the surface weight before densification is determined by contactless measuring methods. Contactless measuring methods, also known as non-tactile measurement methods, rely on measurement sensors without actually touching the object being measured.

Especially, the surface weight before densification is determined by electromagnetic and/or ultrasound measurements, preferably by X-ray radiation, UV radiation, infrared radiation, microwaves, radio waves and/or ultrasound waves.

This has the advantage that the surface weight can be measured in one step, providing a time and /or cost efficient procedure.

The determination by X-ray measurement encompasses the steps of casting beams of X-rays waves over and across the at least one wood layer. As visible light, X-rays loose a certain amount of energy when they pass through different materials. The energy loss depends on the absorption behaviour of the material, i.e. the at least one wood layer.

The reduction of energy is caused by absorption. The intensity of X-rays is weakened according to the mass of the penetrated material, e.g. a wood layer. This weakening allows determining the basis weight of the at least one wood layer. The beams pass through the at least one wood layer to reach a detector and beam energy levels are recorded as numerical values.

Before measurements based on electromagnetic and/or ultrasound waves are conducted, the appropriate measurement device is preferably calibrated. Possible calibration methods include for example placing a calibration object of a known area and weight in a precise position in the electromagnetic and/or ultrasound waves' field of view, take visual images in the form of photograph, film and/or video signals and express the relation between the area and weight of the at least one wood layer and the signal of the electromagnetic and/or ultrasound waves arriving at the detector after passing through the at least one wood layer.

Electromagnetic and/or ultrasound wave measurements are contactless, non-destructive scanning processes and do not alter the at least one wood layer in any way. Because no touch probe device is used, there are no contact forces which could cause distortions or deflections that might affect measurements. Furthermore, electromagnetic and/or ultrasound wave measurements can provide results in less time, with greater ease and in a higher precision in comparison to other measurement methods.

In a further preferred embodiment, the thickness of the at least one wood layer after densification is predicted only by the surface weight of the at least one wood layer before densification and the target density of the at least one wood layer after densification.

This provides a simple and quick way of predicting the thickness of the at least one wood layer after densification. Especially, since the target density of the at least one wood layer is determined in advance, only the surface weight of the at least one wood layer before densification has to be measured to predict the thickness after densification.

However, it is also possible that further parameters may be taken into account to predict the thickness of the at least one wood layer.

In a further special embodiment, the target density of the at least one wood layer after densification is selected in the range of 1'300 - 1'600 $kg/m^3$, preferably 1'350 - 1'500 $kg/m^3$, especially 1'400 $kg/m^3$.

The densification allows for producing highly densified wood layers with a final density of up to 1'600 $kg/m^3$. Thereby, when compared with the wood layer before densification, it is possible to improve the chemical and physical properties, such as the mechanical stability, colour stability against temperature and UV-Vis light, significantly even without addition of synthetic additives. Thus, highly densified and stable wood layers with and without any synthetic components can be produced.

Especially, the density of the at least one wood layer after densification is selected in the range of at least 900 $kg/m^3$, especially at least 1'200 $kg/m^3$, in particular at least 1'300 $kg/m^3$, preferably between 1'300 - 1'600 $kg/m^3$.

These compression degrees typically give rise to significantly enhanced densified materials, especially densified wood materials, which are suitable for a large number of different applications. Nevertheless, other compression degrees might be suitable as well.

Especially, the method is effected such that a tensile strength of the at least one wood layer after densification is 1.5 - 4, especially 2-3, times as high as the tensile strength of the at least one wood layer before densification.

Preferably, the method is effected such that a density of the at least one wood layer after densification is a multiple of raw density of the at least one wood layer before densification, with densification factors of typically 1.01 - 100. The densification factors are in particular 1.1 - 50, especially 1.5 - 20, preferably 2 - 10.

In particular, a joint thickness of at least two wood layers after densification is predicted.

This provides the possibility of predicting the thickness of a stack of wood layers, such as laminated structures, in a quick and easy way and without needing to predict the thickness of individual wood layers separately.

Especially preferred, the thickness of the at least one wood layer, preferably of the at least two wood layers, is predicted for each wood layer separately.

In a further preferred embodiment, the method according to the invention can predict the thickness of at least two wood layers, whereby at least one wood layer is treated with an adhesive, especially a synthetic, natural, fossil or bio-based adhesive, and/or at least one adhesive foil is arranged between the at least two wood layers, such that the at least two wood layers are bonded together upon densification in order to produce a laminated structured.

In a preferred embodiment, the at least one wood layer before densification is sorted into a first category system, in particular before step (i), preferably based on its format, wood type and/or function.

Especially, the at least two wood layers before densification are sorted into a first category system, in particular before step (i), preferably based on their format, wood type and/or function.

Preferably, sorting the at least one wood layer into a first category system is realized by means of an algorithm, especially a sorting algorithm.

Preferably, sorting the at least two wood layers into a first category system is realized by means of an algorithm, especially a sorting algorithm.

Sorting the at least one wood layer, especially the at least two wood layers, into a first category supports the algorithm in selecting the correct wood layers for the densification process and helps minimizing errors in the selection process of picking the adequate wood layers.

However, it is also possible to omit the sorting of the at least one wood layer, especially the at least two wood layers, into a first category system.

In particular, especially after step (i), the at least one wood layer before densification is sorted into a second category system, preferably based on its surface weight.

Preferably, especially after step (i), the at least two wood layers before densification are sorted into a second category system, preferably based on their surface weight.

Especially, sorting the at least one wood layer into a second category system is realized by means of an algorithm, especially a sorting algorithm.

Preferably, sorting the at least two wood layers into a second category system is realized by means of an algorithm, especially a sorting algorithm.

Sorting the at least two wood layers into a second category supports the algorithm in selecting the correct wood layers for the densification process and helps minimizing errors in the selection process of picking the adequate wood layers.

However, it is also possible to omit the sorting the at least one wood layer, especially the at least two wood layers into a second category system.

The term "sorting" in particular means assigning at least one wood layer, especially at least two wood layers, to the first and/or second category system.

In a further preferred embodiment, the at least two wood layers from the second category system are combined based on an algorithm, especially a combining algorithm.

This ensures that appropriate wood layers can be selected to form a stack of wood layers exhibiting the desired characteristics, such as a defined total thickness. Different sorting algorithms may be used such as simple regression algorithms or more complex machine learning algorithms. Examples for suitable algorithms are Ridge, Lasso, Random forest and/or Support Vector.

In another preferred embodiment it is also conceivable that the at least two wood layers are sorted into a first and/or second category system, especially by an algorithm, preferably a sorting algorithm, but only one single wood layer, preferably from the first and/or second category system, undergoes densification.

This ensures that a single wood layer exhibiting the desired characteristics, such as a defined total thickness for example can be selected.

Especially, at least one wood layer is selected from the first and/or second category system based on an algorithm, especially a sorting algorithm.

Preferably, a target thickness of the at least one wood layer after densification is defined.

In a preferred embodiment, the algorithm comprises the steps of:

2a) Sorting the at least one wood layer, especially the at least two wood layers, into a first category system based on their format, wood type and/or function

2b) Sorting the at least one wood layer, especially the at least two wood layers, into a second category system based on their surface weight

2c) Calculating combinatory sets of at least two individual wood layers or selecting one single wood layer from the first and/or second category system which should result in a stack of wood layers or a single wood layer with a predicted thickness

In another preferred embodiment, after step 2c) a further step of comparing the predicted thickness with the actual thickness of the stack of wood layers or the single wood layer is conducted. However, it is conceivable that the at least two wood layers are combined without an algorithm. For example, the wood layers may also be combined manually.

Especially preferred, the at least two combined wood layers are densified.

It is possible that the at least two combined wood layers are densified together or individually.

In another preferred embodiment, the at least two combined wood layers as well as at least one adhesive layer, preferably placed between the at least two combined wood layers, are densified.

Preferably, the adhesive does have a negligible thickness compared to the wood layers.

In another preferred embodiment, the at least two combined wood layers are densified and laminated simultaneously, e.g. in the form of a stack. Thereby, a laminated structure is produced during the densification process.

Especially, the at least two combined wood layers are densified individually and bonded together with an adhesive.

Preferably, a target thickness of the at least two densified wood layers after densification is defined.

This way it is possible to manufacture products, e.g. laminated structures, in various thicknesses, depending on what the thickness requirements are. Furthermore, production waste can be minimized. Also, the production speed can be accelerated since the manufacture process needs less adjustments.

In a preferred embodiment, the target thickness of the at least two densified wood layers including at least one layer of adhesive is defined.

Preferably, the at least one layer of adhesive is placed between the at least two densified wood layers so that the at least two densified wood layers are bonded together.

Especially preferred, an actual thickness of the at least two densified wood layers after densification is measured.

Preferably, an actual thickness of the at least one wood layer after densification is measured.

In another preferred embodiment, an actual thickness of the single wood layer after densification is measured.

Measuring an actual thickness after densification provides means of comparing the predicted thickness of the at least two densified wood layers or the densified single wood layer with an actual resulting thickness. This comparison serves as monitoring mechanism to ensure that the production requirements regarding the targeted thickness are met. Furthermore, it helps in adjusting the densifying process early in the process, which saves time and costs.

Preferably, the step of comparing the predicted thickness with the actual thickness is realized by means of an algorithm, especially a comparing algorithm.

In a preferred embodiment, the sorting algorithm, the combining algorithm and the comparing algorithm are different algorithms.

In another preferred embodiment, the sorting algorithm, the combining algorithm and/or the comparing algorithm are part of the same algorithm.

In a special embodiment, the target thickness of the at least two densified wood layers after densification and the actual thickness of the at least two densified wood layers after densification are compared, especially by a comparing algorithm

Preferably, the target thickness of the at least one wood layer after densification and the actual thickness of the at least one wood layer after densification are compared, especially by a comparing algorithm

In another special embodiment, the target thickness of the single wood layer after densification and the actual thickness of the single wood layer after densification are compared.

This comparison serves as monitoring mechanism to ensure that the production requirements regarding the targeted thickness are met. Furthermore, it helps in adjusting the densifying process early in the process, which saves time and costs.

Preferably, the comparison is realised in an automated manner, especially via a further algorithm.

Most preferably, the algorithm, especially the sorting, combining and/or comparing algorithm, is adjusted depending on the results from the comparison between the target thickness of the at least two densified wood layers or the single wood layer after densification and the actual thickness of the at least two densified wood layers or the single wood layer after densification.

In a further preferred embodiment, an algorithm, especially a sorting, combining and/or comparing algorithm, is adjusted depending on the results from the comparison between the target thickness of the at least one wood layer after densification and the actual thickness of the at least one wood layer after densification

This allows for a quick and easy adjustment of the algorithm, which can lead to a more efficient production procedure.

In a special embodiment, the method for predicting a thickness of at least one wood layer after densification comprises the steps of:

(i0)    Sorting the at least two wood layers into a first category system, preferably based on their format, wood type and/or function

(ii0)    Determining the surface weight of the at least one wood layer before densification

(iii0)    Defining a target density for the at least one wood layer after densification

(iv0)    Predicting the thickness of the at least one wood layer after densification based on the surface weight and the target density

(v0)    Sorting the at least two wood layers into a second category system after step (i0), preferably based on their surface weight

(vi0)    Defining a target thickness of the at least one wood layer after densification

(vii0)    Selecting at least one wood layer from the second category system based on an algorithm

(viii0)    Densifying the at least one wood layer

(ix0)    Optionally, comparing the target thickness of the at least one wood layer after densification and the actual thickness of the at least one wood layer after densification

(continued)

(x0) Optionally, adjusting an algorithm, especially a sorting algorithm, depending on the results from the comparison between the target thickness of the at least one wood layer after densification and the actual thickness of the at least one wood layer after densification

In another preferred embodiment, the method for predicting a thickness of at least one wood layer after densification comprises the steps of:

(i0.5) Sorting the at least two wood layers into a first category system, preferably based on their format, wood type and/or function

(ii0.5) Determining the surface weight of the at least one wood layer before densification

(iii0.5) Defining a target density for the at least one wood layer after densification

(iv0.5) Predicting the thickness of the at least one wood layer after densification based on the surface weight and the target density

(v0.5) Sorting the at least two wood layers into a second category system after step (i0.5), preferably based on their surface weight

(vi0.5) Defining a target thickness of the at least one wood layer after densification

(vii0.5) Selecting at least one wood layer from the second category system based on an algorithm

(viii0.5) Densifying the at least one wood layer

(ix0.5) Comparing the target thickness of the at least one wood layer after densification and the actual thickness of the at least one wood layer after densification

(x0.5) Adjusting an algorithm, especially a sorting algorithm, depending on the results from the comparison between the target thickness of the at least one wood layer after densification and the actual thickness of the at least one wood layer after densification

In another preferred embodiment, the method for predicting a thickness of at least two wood layers after densification comprises the steps of:

(i1) Sorting the at least two wood layers into a first category system, preferably based on their format, wood type and/or function

(ii1) Determining the surface weight of the at least two wood layers before densification

(iii1) Defining a target density for the at least two wood layers after densification

(iv1) Predicting the thickness of the at least two wood layers after densification based on the surface weight and the target density

(v1) Sorting the at least two wood layers into a second category system after step (i1), preferably based on their surface weight

(vi1) Defining a target thickness of the at least two densified wood layers after densification

(vii1) Combining at least two wood layers from the second category system based on an algorithm, in particular a combining algorithm.

(viii1) Densifying the at least two combined wood layers

Especially, the method for predicting a thickness of at least two wood layers after densification comprises the steps of:

(i2) Determining the surface weight of the at least two wood layers before densification

(ii2) Defining a target density for the at least two wood layers after densification

(iii2) Predicting the thickness of the at least two wood layers after densification based on the surface weight and the target density

(iv2) Sorting the at least two wood layers into a first category system after step (i2), preferably based on their surface weight

(v2) Defining a target thickness of the at least two densified wood layers after densification

(continued)

(vi2)   Combining at least two wood layers from the first category system based on an algorithm, in particular a combining algorithm.

(vii2)   Densifying the at least two combined wood layers

Preferably, the method for predicting a thickness of at least two wood layers after densification comprises the steps of:

(i3)   Sorting the at least two wood layers into a first category system, preferably based on their format, wood type and/or function

(ii3)   Determining the surface weight of the at least two wood layers before densification

(iii3)   Defining a target density for the at least two wood layers after densification

(iv3)   Predicting the thickness of the at least two wood layers after densification based on the surface weight and the target density

(v3)   Optionally, sorting the at least two wood layers into a second category system after step (i3), preferably based on their surface weight

(vi3)   Optionally, defining a target thickness of the at least two densified wood layers after densification

(vii3)   Optionally, combining at least two wood layers from the second category system based on an algorithm, in particular a combining algorithm.

(viii3)   Optionally, densifying the at least two combined wood layers

In another preferred embodiment, the method for predicting a thickness of at least one wood layer after densification comprises the steps of:

(i3.1)   Sorting the at least one wood layer into a first category system, preferably based on its format, wood type and/or function

(ii3.1)   Determining the surface weight of the at least one wood layer before densification

(iii3.1)   Defining a target density for the at least one wood layer after densification

(iv3.1)   Predicting the thickness of the at least one wood layer after densification based on the surface weight and the target density

(v3.1)   Sorting the at least one wood layer into a second category system after step (i3.1), preferably based on its surface weight

(vi3.1)   Selecting at least one wood layer from the second category system based on an algorithm, in particular a sorting algorithm

(vii3.1)   Defining a target thickness of the at least one wood layer after densification

(viii3.1)   Densifying the at least one wood layer

(ix3.1)   Optionally, comparing the target thickness of the at least one wood layer after densification and the actual thickness of the at least one wood layer after densification, especially by a comparing algorithm

(x3.1)   Optionally, adjusting an algorithm, especially a sorting, combining and/or comparing algorithm, depending on the results from the comparison between the target thickness of the at least one wood layer after densification and the actual thickness of the at least one wood layer after densification

In another preferred embodiment, the method for predicting a thickness of at least two wood layers after densification comprises the steps of:

(i3.2)   Sorting the at least two wood layers into a first category system, preferably based on their format, wood type and/or function

(ii3.2)   Determining the surface weight of the at least two wood layers before densification

(iii3.2)   Defining a target density for the at least two wood layers after densification

(continued)

(iv3.2)  Predicting the thickness of the at least two wood layers after densification based on the surface weight and the target density

(v3.2)  Sorting the at least two wood layers into a second category system after step (i3.2), preferably based on their surface weight

(vi3.2)  Selecting at least two wood layers from the second category system based on an algorithm, in particular a sorting algorithm

(vii3.2)  Defining a target thickness of the at least two wood layers after densification

(viii3.2)  Densifying the at least two wood layers

(ix3.2)  Optionally, comparing the target thickness of the at least two wood layers after densification and the actual thickness of the at least two wood layers after densification, especially by a comparing algorithm

(x3.2)  Optionally, adjusting an algorithm, especially a sorting, combining and/or comparing algorithm, depending on the results from the comparison between the target thickness of the at least two wood layers after densification and the actual thickness of the at least two wood layers after densification

A second aspect of the invention refers to a method for producing at least one densified wood layer comprising the steps of:

(i4)  Providing at least one wood layer

(ii4)  Predicting the thickness of the at least one wood layer from step (i4) according to the method described in the present description

(iii4)  Densifying the at least one wood layer

Densification can be effected as described above.

Especially, the method for producing at least one densified wood layer may comprise the steps of:

(i4.5)  Providing at least two wood layers

(ii4.5)  Predicting the thickness of the at least two wood layers from step (i4.5) according to the method described in the present description

(iii4.5)  Densifying the at least two wood layers

In a preferred embodiment, the predicted thickness from step (ii4) is compared with the actual thickness of the at least one wood layer after step (iii4) and/or the predicted thickness from step (ii4.5) is compared with the actual thickness of the at least one wood layer after step (iii4.5).

A third aspect of the invention is concerned with a method for producing a laminated structure comprising the steps of:

(i5)  Providing at least two densified wood layers produced according to the method described above

(ii5)  Laminating the at least two densified wood layers from step (i5)

Preferably, the at least two wood densified wood layers of the laminated structure are firmly bonded, e.g. with an adhesive and/or an adhesive foil, after step (i5).

Especially, the laminating process is conducted according to the method described above.

In a preferred embodiment, after step (ii5) additional layers can be added to the at least two laminated densified wood layers.

Especially, the additional layers are of non-wooden materials and/or have a defined thickness.

Preferably, the additional layers are not densified during the densifying process described above.

In another preferred embodiment, first, the at least two wood layers are sorted into a first category system and subsequently, the at least two wood layers are sorted into a second category system.

Other advantageous embodiments and combinations of features come out from the detailed description below and the totality of the claims.

## Brief description of the drawings

The drawings used to explain the embodiments show:

**Fig. 1** shows a schematic representation of the method according to the invention for predicting a thickness of at least one wood layer after densification.

**Fig. 2** shows a schematic representation of the method according to the invention for predicting a thickness of at least two wood layers after densification.

## Exemplary embodiments

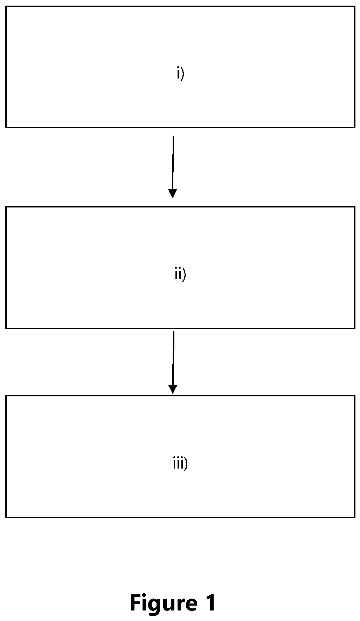

Fig. 1 shows a schematic representation of the method according to the invention for predicting a thickness of at least one wood layer after densification comprising the steps of:

(i) Determining the surface weight of the at least one wood layer before densification

(ii) Defining a target density for the at least one wood layer after densification

(iii) Predicting the thickness of the at least one wood layer after densification based on the surface weight and the target density

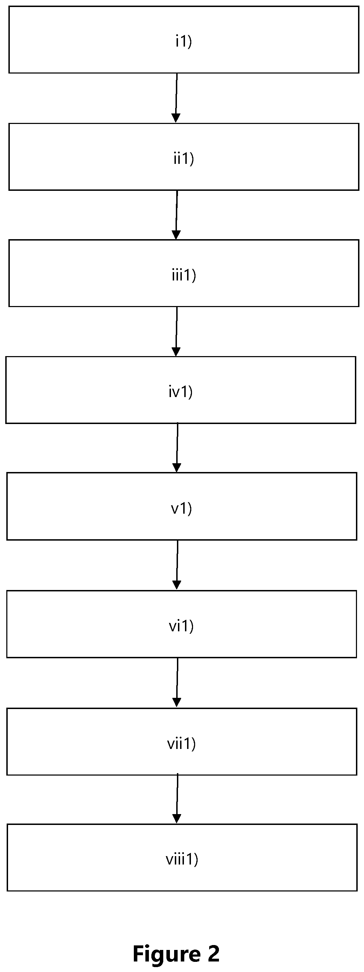

Fig. 2 shows a schematic representation of the method according to the invention for predicting a thickness of at least two wood layers after densification comprising the steps of:

(i1)     Sorting the at least two wood layers into a first category system, preferably based on their format, wood type and/or function
(ii1)    Determining the surface weight of the at least two wood layers before densification
(iii1)   Defining a target density for the at least two wood layers after densification
(iv1)    Predicting the thickness of the at least two wood layers after densification based on the surface weight and the target density

(v1)     Sorting the at least two wood layers into a second category system after step i1), preferably based on their surface weight
(vi1)    Defining a target thickness of the at least two densified wood layers after densification
(vii1)   Combining at least two wood layers from the second category system based on an algorithm
(viii1)  Densifying the at least two combined wood layers

An exemplary study has been carried out as follows:
The study has been conducted with a sample size of 1'200 cards.
Based on known state of the art methods and considering a final thickness of the laminated structure requirement of +/- 5 %, a reject rate of about 50 % of the final laminated structure was expected without sorting prior to the densification and/or lamination. This means 50 % of the laminated structure would be outside the required thickness range.
For the study, different sorting parameters were used to test the resulting production waste. The results were as follows:

I) Using only the thickness of the wood layer as sorting parameter, the production waste amounted to approx. 25 - 30 %

II) Using the thickness and the target density of the wood layer, the production waste amounted to approx. 15 - 20 %.

III) Using only the surface weight as sorting parameter, the production waste amounted to less than 10 %.

When using the thickness and the target density as sorting parameters, 4 measurements are needed to predict the final thickness of the wood layer(s), i.e. thickness, length, width and weight. With the method according to the invention, only 3 measurements are necessary for a better prediction. Hence, the variability is reduced and the prediction is improved. Furthermore, the production waste can be minimized with a simple sorting method according to the invention which can be implemented in a cost-efficient manner.

**Claims**

1. Method for predicting a thickness of at least one wood layer after densification comprising the steps of:

   (i) Determining the surface weight of the at least one wood layer before densification
   (ii) Defining a target density for the at least one wood layer after densification
   (iii) Predicting the thickness of the at least one wood layer after densification based on the surface weight and the target density

2. Method according to claim 1, wherein the surface weight is determined by weighing the at least one wood layer and measuring the area of the at least one wood layer, both before densification.

3. Method according to claim 2, wherein the area of the at least one wood layer before densification is measured with a camera.

4. Method according to any of the aforementioned claims, wherein the surface weight before densification is determined by electromagnetic and/or ultrasound measurements, preferably by X-ray radiation, UV radiation, infrared radiation, microwaves, radio waves and/or ultrasound waves.

5. Method according to any of the aforementioned claims, wherein the thickness of the at least one wood layer after densification is predicted only by the surface weight of the at least one wood layer before densification and the target density of the at least one wood layer after densification.

6. Method according to any one of the aforementioned claims, wherein the target density of the at least one wood layer after densification is selected in the range of 1'300 - 1'600 $kg/m^3$, preferably 1'350 - 1'500 $kg/m^3$, especially 1'400 $kg/m^3$.

7. Method according to any of the aforementioned claims, wherein the at least one wood layer before densification is densified.

8. Method according to any of the aforementioned claims, wherein a target thickness of the at least one wood layer after densification is defined.

9. Method according to any of the aforementioned claims, wherein an actual thickness of the at least one wood layer after densification is measured.

10. Method according to claims 8 and 9, wherein the target thickness of the at least one wood layer after densification and the actual thickness of the at least one wood layer after densification are compared.

11. Method according to claim 10, wherein an algorithm, especially a sorting, combining and/or comparing algorithm, is adjusted depending on the results from the comparison between the target thickness of the at least one wood layer after densification and the actual thickness of the at least one wood layer after densification.

12. Method according to any of the aforementioned claims, wherein at least two wood layers before densification are sorted into a first category system, in particular before step (i), preferably based on their format, wood type and/or function.

13. Method according to any of the aforementioned claims, wherein after step (i) the at least two wood layers before densification are sorted into a second category system, preferably based on their surface weight.

14. Method according to claim 12 or 13, wherein at least one wood layer is selected from the first and/or second category system based on an algorithm, especially a sorting algorithm.

15. Method according to claim 13, wherein the at least two wood layers from the second category system are combined based on an algorithm, especially a combining algorithm.

16. Method for predicting a thickness of at least one wood layer after densification comprising the steps according to claims 1, 7, 8, 12, 13, 14 and optionally claims 10 and/or 11.

17. Method for producing at least one densified wood layer comprising the steps of:

   (i4) Providing at least one wood layer
   (ii4) Predicting the thickness of the at least one wood layer from step (i4) according to any of the claims 1 - 6 and 8-15
   (iii4) Densifying the at least one wood layer

18. Method for producing a laminated structure comprising the steps of:

   (i5) Providing at least two densified wood layers produced according to claim 17
   (ii5) Laminating the at least two densified wood layers from step (i5)

**Figure 1**

```
┌─────────────────────────────────┐
│                                 │
│              i1)                │
│                                 │
└─────────────────────────────────┘
                 │
                 ▼
┌─────────────────────────────────┐
│                                 │
│              ii1)               │
│                                 │
└─────────────────────────────────┘
                 │
                 ▼
┌─────────────────────────────────┐
│                                 │
│              iii1)              │
│                                 │
└─────────────────────────────────┘
                 │
                 ▼
┌─────────────────────────────────┐
│                                 │
│              iv1)               │
│                                 │
└─────────────────────────────────┘
                 │
                 ▼
┌─────────────────────────────────┐
│                                 │
│              v1)                │
│                                 │
└─────────────────────────────────┘
                 │
                 ▼
┌─────────────────────────────────┐
│                                 │
│              vi1)               │
│                                 │
└─────────────────────────────────┘
                 │
                 ▼
┌─────────────────────────────────┐
│                                 │
│              vii1)              │
│                                 │
└─────────────────────────────────┘
                 │
                 ▼
┌─────────────────────────────────┐
│                                 │
│              viii1)             │
│                                 │
└─────────────────────────────────┘
```

**Figure 2**

Europäisches
Patentamt
European
Patent Office
Office européen
des brevets

# EUROPEAN SEARCH REPORT

**Application Number**

EP 24 20 2322

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 107 322 724 A (WANG KE) 7 November 2017 (2017-11-07) * abstract * | 1-18 | INV. G06Q10/04 G01B21/08 G01N33/46 |
| A | WO 2019/235503 A1 (GOTO MOKUZAI KK [JP]) 12 December 2019 (2019-12-12) * abstract * | 1-18 | |
| A | CABRAL JOHN PAUL ET AL: "Densification of timber: a review on the process, material properties, and application", JOURNAL OF WOOD SCIENCE, SPRINGER SINGAPORE, SINGAPORE, vol. 68, no. 1, 30 March 2022 (2022-03-30) , XP037774789, ISSN: 1435-0211, DOI: 10.1186/S10086-022-02028-3 [retrieved on 2022-03-30] * abstract * | 1-18 | |
| A | CN 109 623 999 A (UNIV SOUTH CHINA AGRICULT) 16 April 2019 (2019-04-16) * abstract * | 1 | **TECHNICAL FIELDS SEARCHED (IPC)** G06Q G01B G01N |

The present search report has been drawn up for all claims

| Place of search | Date of completion of the search | Examiner |
|---|---|---|
| Munich | 28 February 2025 | Dedek, Frédéric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 20 2322

This annex lists the patent family members relating to the patent documents cited in the above-mentioned European search report.
The members are as contained in the European Patent Office EDP file on
The European Patent Office is in no way liable for these particulars which are merely given for the purpose of information.

28-02-2025

| Patent document cited in search report | | Publication date | Patent family member(s) | | Publication date |
|---|---|---|---|---|---|
| CN 107322724 | A | 07-11-2017 | NONE | | |
| WO 2019235503 | A1 | 12-12-2019 | JP | 6450489 B1 | 09-01-2019 |
| | | | JP | 2019209590 A | 12-12-2019 |
| | | | WO | 2019235503 A1 | 12-12-2019 |
| CN 109623999 | A | 16-04-2019 | NONE | | |

EPO FORM P0459

For more details about this annex : see Official Journal of the European Patent Office, No. 12/82